## Europäisches Patentamt

## European Patent Office

## Office européen des brevets

(19)

(11) Veröffentlichungsnummer: **0 132 732**
**B1**

(12) **EUROPÄISCHE PATENTSCHRIFT**

(45) Veröffentlichungstag der Patentschrift:
08.06.88

(51) Int. Cl.⁴: **C 07 K 7/10, A 61 K 37/02**

(21) Anmeldenummer: **84108293.6**

(22) Anmeldetag: **14.07.84**

(54) **Homologe des Aprotinin mit anderen Aminosäuren in Position 15 anstelle von Lysin, Verfahren zu ihrer Herstellung sowie ihre Verwendung als Arzneimittel.**

(30) Priorität: 28.07.83 DE 3327277
03.11.83 DE 3339693

(43) Veröffentlichungstag der Anmeldung:
13.02.85 Patentblatt 85/7

(45) Bekanntmachung des Hinweises auf die Patenterteilung:
08.06.88 Patentblatt 88/23

(84) Benannte Vertragsstaaten:
AT BE CH DE FR GB IT LI NL SE

(56) Entgegenhaltungen:
CHEMICAL ABSTRACTS, Band 97, 1982, Seite 900, Nr. 216639r, Columbus, Ohio, US; I. STOINEVA et al.: "Semisynthesis of (Phe15-NHC6H4NO2)-aprotinin(15,16)-glycinamide", & AGENTS ACTIONS SUPPL. 1982, AAS 9(RECENT PROG. KININS), 195-200
H. Jering et al. Eur. J. Biochem. 61, 443-452 (1976)
H.R. Wenzel et al. Angew. Chem. 93 (1981) No. 3, 292-293

(73) Patentinhaber: **BAYER AG, Konzernverwaltung RP Patentabteilung, D-5090 Leverkusen 1 Bayerwerk (DE)**

(72) Erfinder: **Tschesche, Harald, Prof. Dr., Im Strohsiek 24, D-4800 Bielefeld 1 (DE)**
Erfinder: **Wenzel, Herbert, Dr., Gracht 11, D-4330 Mülheim 1 (DE)**
Erfinder: **Schmuck, Rainer, Dr., Schlosshofstrasse 184, D-4800 Bielefeld 1 (DE)**
Erfinder: **Schnabel, Eugen, Dr., Schimmelweg 6, D-5600 Wuppertal 11 (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

LIBER, STOCKHOLM 1988

**Beschreibung**

**Homologe des Aprotinin mit anderen Aminosäuren in Position 15 anstelle von Lysin, Verfahren zu ihrer Herstellung sowie ihre Verwendung als Arzneimittel**

Aprotinin ist ein bekannter Kallikrein-Trypsin-Inhibitor aus Rinderorganen. Seine Struktur ist ebenfalls bekannt (Formel (I). Im reaktiven Zentrum der Peptidkette befindet sich in Position 15 ein Lysinrest, der für die Spezifität des Inhibitors entscheidend ist.

(I)

Die vorliegende Erfindung betrifft nun neue Homologe des Aprotinin in welchen dieser Lysinrest ausgetauscht ist gegen Glycin, L-Alanin, L-Valin, L-Leucin, L-Isoleucin, L-Methionin, L-Arginin oder L-Norleucin, L-Norvalin, L-α-Aminobuttersäure, Dehydroalanin oder L-Homoserin. Die Erfindung betrifft weiter ein Verfahren zur Herstellung dieser Homologen, dabei auftretende Zwischenprodukte sowie ihre Verwendung als Arzneimittel.

Aus H. Jering und H. Tschesche, Europ. J. Biochem. 61, 443 (1976) ist bekannt, daß in das durch eine Folge von chemischen und enzymatischen Reaktionen darstellbare Des-Lys[15]-Aprotinin* anstelle des fehlenden Lysins mit Hilfe von Enzymen basische und aromatische Aminosäuren eingebaut werden können, während auf diesem Wege die Inkorporation anderer Aminosäuren in Pos. 15 nicht möglich war. Als Aprotinin* wird im folgenden Aprotinin bezeichnet, bei dem die Peptidbindung zwischen Lysin 15 und Alanin 16 hydrolysiert ist.

Das als enzymatische Mutation bezeichnete Verfahren, nach dem die Aprotinin-Homologen mit Phenylalanin, Tryptophan und Arginin hergestellt wurden, liefert nur für die Homologen mit Phenylalanin und Tryptophan Einkettenproteine. Das Arginin[15]-derivat wird nur als Zweikettenprotein mit einer Spaltung der Bindung Arginin 39 - Alanin 40 und Verlust des Arginin 39 gewonnen.

Bei der als chemische Mutation bezeichneten und mit Hilfe von wasserlöslichen Carbodiimiden durchgeführten Kondensation von Des-Lys[15]-Aprotinin* mit den Estern der Aminosäuren Glycin, L-Alanin, L-Valin, L-Leucin, L-Methionin und L-Arginin werden Aprotinin*-Derivate erhalten, die in Pos. 15 teilweise den betreffenden Aminosäureester tragen, bei denen sich dieser Aminosäureester aber gleichzeitig an alle oder einen Teil der peripheren Carboxylgruppen in den Seitenketten der Asparaginsäurereste (Pos. 3 und 50) sowie der Glutaminsäurereste (Pos. 7 und 49) und des terminalen Alanin (Pos. 58) unter Bildung von Amidbindungen ankondensiert [H.R. Wenzel und R. Tschesche, Angw. Chem. 93, 292 (1981)]. Außerdem entstehen während der Umsetzungen durch Anlagerungen der Carbodiimide an die Carboxylgruppen unerwünschte Acylharnstoffe. Nach enzymatischer Resynthese der Peptidbindung zwischen dem Aminosäureester in Pos. 15 und Alanin 16 werden daher gemäß dieser Literaturstelle keine reinen Aprotinin-Homologen erhalten, sondern Gemische von Derivaten dieser Homologen. Reine Aprotinin-Homologe, die in Pos. 15 andere Aminosäuren als Phenylalanin und Trypeophan enthalten, sind daher noch nicht bekannt.

Die vorliegende Erfindung stellt nun solche Homologen zur Verfügung. Diese Homologen unterscheiden sich vom Aprotinin allein dadurch, daß sie anstelle von Lysin in Pos. 15 Glycin, L-Alanin, L-Valin, L-Leucin, L-Isoleucin, L-Methionin, L-Arginin, L-α-Aminobuttersäure, L-Norvalin, L-Norleucin, Dehydroalanin oder L-Homoserin tragen. Diese Homologen können in einer Folge geeigneter chemischer und enzymatischer Reaktionsschritte in guten Ausbeuten hergestellt werden.

Dazu wird zunächst modifiziertes Aprotinin mit der zwischen Lysin[15] und Alanin[16] gespaltenen Peptidbindung (= Aprotinin*) in an sich bekannter Weise hergestellt. Die Herstellung von Aprotinin* kann auch ohne vorherige Reduktion der Disulfidbrücke Cys[14]-Cys[38] mit den Enzymen Plasmin oder Seesterntrypsin aus Dermasterias imbricata durchgeführt werden. Dann werden die sechs freien Carboxylgruppen des Moleküls in

bekannter Weise mit Hilfe von methanolischer Salzsäure verestert unter Bildung des Aprotinin*-hexamethylesters. Die Veresterung kann aber auch in Analogie zu bekannten Verfahren mit Trialkyloxoniumfluoroborat vorgenommen werden. Des weiteren eignet sich die Umsetzung von Aprotinin* mit Methanol-Thionylchlorid zur Herstellung von Aprotinin*-hexamethylester. Zur Veresterung sind anstelle von Methanol auch andere Alkohole mit geringer proteindenaturierender Wirkung geeignet wie beispielsweise Ethanol sowie aliphatische Alkohole mit bis zu 6 C-Atomen, die gegebenenfalls auch Substituenten tragen können. Bevorzugt wird jedoch der Hexamethylester von Aprotinin* für die weiteren Umsetzungen verwendet. Im Bedarfsfall können in der Rohsubstanz infolge unvollständiger Umsetzung oder infolge von Nebenreaktionen entstandene Begleitsubstanzen in an sich bekannter Weise durch Ionenaustauschchromatographie an Kationenaustauschern abgetrennt werden. Hierfür eignen sich Carboxyl- oder Sulfo-Gruppen tragende Kationenaustauscher, wie CM-Cellulose, CM-Sephadex, CM-Sepharose und SP-Sephadex. Die Ionenaustauschchromatographie wird bei pH-Werten zwischen 3 und 7, vorzugsweise bei pH 4,0 bis 6,0, mit geeigneten Pufferlösungen durchgeführt. Derartige Pufferlösungen sind beispielsweise Phosphat-, Acetat-, Succinat- oder Citratlösungen, deren Salzgehalt während der Elution kontinuierlich oder diskontinuierlich erhöht wird. Vorzugsweise wird Natriumchlorid für die Erzeugung des Salzgradienten verwendet.

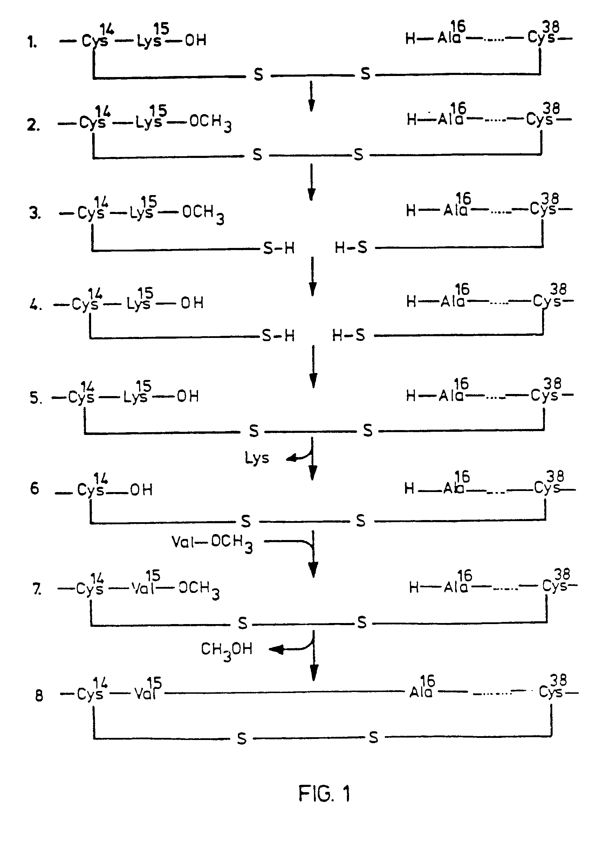

Ausgehend vom Aprotinin* ist beispielhaft die Synthese von Valin-15-Aprotinin in Fig. 1 schematisch wiedergegeben. In dem dargestellten Syntheseschema beschränkt sich die Wiedergabe der Reaktionsschritte auf den das aktive Zentrum (Pos. 15) betreffenden Teil des Aprotininmoleküls. Alle die peripheren Carboxylgruppen erfassenden Reaktionen (Veresterungen und Verseifungen) werden im Formelschema nicht berücksichtigt.

Die Substanzen werden wie folgt bezeichnet:
1) Aprotinin*
2) Aprotinin*-hexamethylester
3) Di-Cysteinyl-14,38-Aprotinin*-hexamethylester
4) Di-Cysteinyl-14,38-Aprotinin*-pentamethylester
5) Aprotinin*-pentamethylester
6) Des-Lysin-15-Aprotinin*-pentamethylester
7) L-Valin-15-Aprotinin*-hexamethylester
8) L-Valin-15-Aprotinin

Im Di-Cysteinyl-14,38-Aprotinin*-hexamethylester und im Di-Cysteinyl-14,38-Aprotinin*-pentamethylester sind die zwischen Pos. 15 und 16 gespaltenen Peptidketten noch kovalent über die Disulfidbrücken der Halbcystine 5/55 bzw. 30/51 verknüpft, was in dem Formelschema nicht erkennbar ist.

Zur selektiven Freisetzung der Carboxylgruppe von Lysin 15 wird im Aprotinin*-hexamethylester zunächst in einem ersten Reaktionsschritt mit Mercaptoethanol oder vorzugsweise mit Dithioerythrit in an sich bekannter Weise die exponierte Disulfidbrücke zwischen den Cysteinresten in den Pos. 14 und 38 selektiv reduziert.

Dazu können 5-100 Mole Dithioerythrit je Mol Aprotinin*-hexamethylester eingesetzt werden, vorzugsweise etwa 15 Mole.

Die Reduktion wird bei pH-Werten von 3 bis 7,5, vorzugsweise bei pH 4,0-6,5 in wäßrigen Pufferlösungen durchgeführt. Bevorzugte Puffer sind beispielsweise Succinat-, Phosphat- oder Acetat-puffer, die 0,005-0,5 M sein können, vorzugsweise jedoch 0,1 M. Zum Abtrennen von überschüssigem Dithioerythrit wird die Reaktionslösung direkt oder nach dem Einengen über eine mit einem geeigneten Molekularsieb gefüllte Chromatographiesäule filtriert. Als Elutionsmittel sind flüchtige Puffer mit pH-Werten von 2-6,5 brauchbar, vorzugsweise wird jedoch 0,05-0,1 M Essigsäure als Lösungsmittel verwendet. Man kann aber die Freisetzung der Carboxylgruppe des Lysinrestes 15 auch ohne vorherige Abtrennung des Dithioerythrit vornehmen.

Die selektive Verseifung des Hexamethylesters wird vorteilhaft ohne vorherige Reduktion der Disulfidbrücke Cys 14-38 durchgeführt. Es entfällt dann der Oxidationsschritt vor der Zugabe der Carboxypeptidase. Zur selektiven Verseifung der Estergruppe am Lysinrest 15 des reduzierten Aprotinin*-hexamethylester wird mit an den Tryptophanresten formyliertem Trypsin umgesetzt, das keine amidolytische wohl aber nach entsprechender Aktivierung noch hohe esterolytische Aktivität mit Trypsinspezifität besitzt. Die selektive Verseifung wird in geeigneten Pufferlösungen durchgeführt. Geeignete Puffer hierfür sind insbesondere Phosphat-, Succinat-, Acetat- oder Citrat-Puffer. Sie können 0,01-0,5 M sein, vorzugsweise 0,1 M. Die bevorzugte Umsetzungstemperatur ist 25-37°C, jedoch ist die Hydrolyse allgemein bei Temperaturen von 4-50°C durchführbar. Die Reaktionszeiten liegen zwischen 1 und 48 Stunden, vorzugsweise 2 - 10 Stunden.

Die selektive Hydrolyse des Lysin 15-Esters ist bei Zusatz von organischen Lösungsmitteln zu der Reaktionslösung auch mit unmodifiziertem Trypsin möglich, da die proteolytische Aktivität von Trypsin durch organische Lösungsmittel wie Dioxan oder Formamid und 2-Chlorethanol ebenfalls unterdrückt wird. Für die Umsetzungen eignen sich insbesondere wässrige Pufferlösungen, die mit Dioxan verdünnt werden. Dabei wird ein Dioxangehalt zwischen 30 und 75 Vol-prozent, vorzugsweise 40-66 Vol-prozent gewählt. Als bevorzugte Pufferlösungen seien insbesondere Acetat-, Succinat- oder Phosphatpuffer genannt. Ihre Molarität kann 0,01-0,5 betragen, vorzugsweise 0,05-0,2 M, und ihr pH-Wert zwischen 2 und 7, vorzugsweise zwischen 3,0 und 6,5 liegen.

Für die selektive Esterhydrolyse werden 0,1-30 Molprozent Trypsin verwendet, vorzugsweise 1 - 10 Molprozent. Je nach Enzymeinsatz und dem pH-Wert der Pufferlösung beträgt die Reaktionszeit 0,1-10 Stunden. Für die selektive Verseifung kann der Aprotinin*-hexamethylester vorteilhaft direkt oder nach Reduktion der Cys 14-38-Disulfidbrücke verwendet werden.

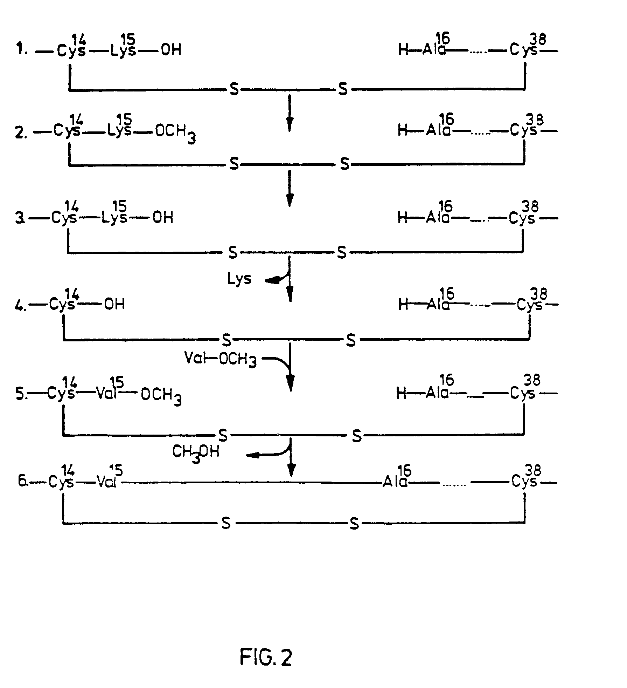

In Fig. 2 ist der Syntheseweg für den Einbau von Valin in Pos. 15 bei Verwendung von Aprotinin*-hexamethylester schematisch wiedergegeben. Wie in Abb. 1 beschränkt sich die Wiedergabe auf den das aktive Zentrum (Pos. 15) betreffenden Teil des Aprotininmoleküls; die die peripheren Carboxylgruppen betreffenden Umsetzungen werden im Formelschema nicht berücksichtigt.

Die Substanzen werden wie folgt bezeichnet:

1. Aprotinin*
2. Aprotinin*-hexamethylester
3. Aprotinin*-pentamethylester
4. Des-Lysin-15-Aprotinin*-pentamethylester
5. L-Valin-15-Aprotinin*-hexamethylester
6. L-Valin-15-Aprotinin

Nach der selektiven Verseifung am Lysin 15 werden der Cys 14-38 reduzierte Aprotinin*-pentamethylester bzw. Aprotinin*-pentamethylester sowie unverseifte Ausgangssubstanz und infolge weitergehender Hydrolyse gebildete stärker saure Komponenten und modifiziertes Trypsin bzw. Trypsin nach in der Proteinchemie üblichen Methode getrennt. Als derartige Verfahren seien hier die Fällung von Trypsin bzw. modifiziertem Trypsin mit Perchlorsäure oder vorzugsweise Trichloressigsäure erwähnt sowie die Filtration über mit geeigneten Molekularsieben gefüllte Chromatographiesäulen, wobei wegen der noch vorhandenen antitryptischen Aktivität des Aprotinin*-Derivates als Elutionsmittel Lösungen mit pH-Werten unterhalb 7,0 vorzugsweise zwischen pH 1,5 und 3,5 verwendet werden. Ein bevorzugtes Lösungsmittel ist 0,05-0,1 M Essigsäure, deren pH-Wert mit Salzsäure auf den gewünschten Wert eingestellt wird. Die bei der Gelfiltration anfallende Inhibitorfraktion aus unverseiftem Aprotinin*-hexamethylester, dem gewünschten Pentamethylester und weitergehend verseiften Derivaten kann ggf. durch Ionenaustauschchromatographie fraktioniert werden. Die Fraktionierung wird unter analogen Bedingungen durchgeführt, wie vorausstehend (und ausführlich in Beispiel 1) für Aprotinin*-hexamethylester beschrieben.

Beim Einsatz von Aprotinin*-hexamethylester mit reduziertem Cys 14-38-Disulfid wird vor dem Abspalten des Lysinrestes 15 mit Carboxypeptidase die Disulfidbrücke zwischen Cystein 14 und 38 oxidativ geschlossen. Diese Oxidation wird bei pH-Werten von 3,0-9,0, vorzugsweise bei pH 4,5-7,0 durch Oxidation im Luftstrom durchgeführt und der Reaktionsverlauf anhand der SH-Werte nach Ellman kontrolliert.

Die Abspaltung von Lysin 15 aus dem Aprotinin*-pentamethylester gelingt glatt mit Carboxypeptidasen, wie z. B. Carboxypeptidase Y, besonders bequem jedoch mit Carboxypeptidase B in gepufferten Lösungen bei pH-Werten zwischen 3 und 7,5, vorzugsweise bei pH 4,0-7,0. Carboxypeptidase und Des-Lys[15] Aprotinin*-pentamethylester werden nach dem Ansäuern der Reaktionslösung in an sich bekannter Weise durch Filtration über Molekularsiebsäulen unter Verwendung von Puffern mit pH-Werten von 1-7, vorzugsweise 2-5, insbesondere jedoch verdünnter Essigsäure als Elutionsmittel getrennt. Der Des-Lysin[15]-Aprotinin*-pentamethylester ist die Ausgangssubstanz für die Synthese der Aprotinin-Homologen und wird beim Gefriertrocknen der betreffenden Filtrate als farblose Substanz erhalten.

Die Einführung der den Lysinrest 15 ersetzenden neuen Aminosäure erfolgt durch Carbodiimid-vermittelte Kondensation des Des-Lysin[15]-Aprotinin*-pentamethylester mit einem Ester der betreffenden Aminosäure in wäßrigen Lösungen, die jedoch auch Zusätze von organischen Lösungsmitteln wie Alkohole, Dimethylformamid oder Dimethylsulfoxid und/oder Salze enthalten können.

Für die Kondensation sind insbesondere wasserlösliche Carbodiimide geeignet wie:

N-Cyclohexyl-N'-2-(4-morpholinyl)-ethyl-carbodiimid-methyl-toluol-4-sulfonat, N-tert.-Butyl-N'-(3-dimethyl-aminopropyl)-carbodiimid-hydrochlorid, N-Cyclohexyl-N'-(3-dimethylaminopropyl)-carbodiimid-hydrochlorid, N-Isopropyl-N'-(3-dimethylaminopropyl)-carbodiimid-hydrochlorid oder vorzugsweise N-Ethyl-N'-(3-dimethylaminopropyl)-carbodiimid-hydrochlorid sowie Gemische von zwei oder mehr der aufgeführten Carbodiimide.

Die Umsetzungen mit den Carbodiimiden werden bei Temperaturen von -10°C bis +35°C durchgeführt, insbesondere zwischen 0°C und 25°C. Dabei wird in der Reaktionslösung durch Zugabe von verdünnter Mineralsäure während der Umsetzung ein pH-Wert aufrechterhalten, der zwischen 4,0 und 7,5 liegt.

In den für die Synthese der Aprotinin-Homologen verwendeten Aminosäurealkylestern kann die Esteralkylgruppe geradkettig, verzweigt oder auch cyclisch sein und bis zu 6 C-Atomen enthalten. Auch Thioester und Phenylester sind brauchbar, doch werden vorzugsweise die Methylester der betreffenden Aminosäuren eingesetzt.

Bei den Kondensationen werden je Mol Des-Lysin-15-Aprotinin*-pentamethylester etwa 10-1000 Mole Aminosäureester und etwa 8-800 Mole Carbodiimid eingesetzt. Dabei entstehen durch Addition der Carbodiimide an die freie Carboxylgruppe des Halbcystinrestes in Pos. 14 als Nebenprodukte wechselnde Mengen an Acylharnstoff (Des-Lys[15]-Cys[14]-Ureido-Aprotinin*-pentamethylester). Außerdem entstehen infolge Addition der Carbodiimide an die phenolischen Hydroxylgruppen der Tyrosinreste O-Arylisoharnstoffderivate. Diese Verbindungen werden gemeinsam mit den Aminosäure-15-Aprotinin*-hexamethylestern bei der Gelfiltration der Reaktionsgemische erhalten und, wie später beschrieben, aus dem Gemisch abgetrennt bzw. freigesetzt.

Die Aminosäure-15-Aprotinin*-hexamethylester sind inhibitorisch aktiv. Sie werden mit 50-95 % Ausbeute gebildet. Diese Ester sind ein weiterer Gegenstand der Erfindung.

Die enzymatisch gesteuerte intramolekulare Amidsynthese wird bei dem erfindungsgemäßen Verfahren dadurch begünstigt, daß die Carboxylgruppe der Aminosäure in Pos. 15 durch die Veresterung aktiviert ist und nicht dissoziiert vorliegt. Die der Resynthese vorgeschaltete Bildung des Acylenzym wird durch eine Umesterung erreicht, die energetisch günstiger ist und vollständiger und ca. $10^3$-fach rascher verläuft als die enzymatische Kondensation mit den in Pos. 15 eine freie Carboxylgruppe enthaltenden Homologen. Für die Synthese ist weiterhin wichtig, daß durch die Bildung des Enzym-Inhibitor-Komplexes an der Aminosäure in Pos. 15 durch eine gewisse tetraedrische Deformation die sterischen Voraussetzungen für eine intramolekulare Resynthese der Peptidbindung geschaffen werden.

Für die enzymatische Knüpfung der Peptidbindung zwischen der neu eingeführten Aminosäure in Pos. 15 und Alanin in Pos. 16 sind insbesondere diejenigen Proteasen brauchbar, in deren Spezifitätstasche die Seitenkette des Aminosäurerestes in Pos. 15 paßt. Für das L-Methionin-enthaltende Homologe sind dies z. B. entweder Chymotrypsin oder Kathepsin G; für die Homologen mit L-Leucin und L-Norleucin sowie L-Norvalin, Chymotrypsin, Kathepsin G oder Elastase aus Granulozyten bzw. Pankreas, für die Homologen mit L-Valin und L-α-Aminobuttersäure Granulozytenelastase und überraschenderweise auch Trypsin. Analog gelingt die Knüpfung der kovalenten Amidbindung bei den Homologen mit L-Alanin durch Pankreaselastase oder Trypsin. Trypsin eignet sich auch für die Synthese der Aprotinin-Homologen, die in Pos. 15 L-Arginin oder überraschenderweise Glycin enthalten.

Die enzymkatalysierte Synthese der Peptidbindung bietet gegenüber den peptidchemischen Verfahren den Vorteil, daß die Bildung unerwünschter Nebenprodukte durch intra- oder intermolekulare Kondensation vermieden wird. Sie kann entweder mit den gelösten Enzymen oder vorteilhaft mit trägergebundenen Enzymen in heterogener Phase vorgenommen werden.

Geeignete Medien für die enzymatische Kondensation sind Pufferlösungen mit pH-Werten zwischen 2 und 10. Vorzugsweise werden Phosphat-, Tris-(hydroxymethyl)-aminomethan-, Borat-Puffer oder Ethanolamin-Puffer mit pH-Werten zwischen 5 und 8,5 verwendet.

Anstelle der kinetisch determinierten Synthesen mit stöchiometrischen Mengen an Proteinasen unter Ausbildung der 1 : 1-Komplexe und ihrer raschen anschließenden Dissoziation zu Enzym und Aprotinin-Homologen, kann die Synthese auch thermodynamisch kontrolliert mit katalytischen Mengen an Enzym durchgeführt werden.

Die Ausbeuten an den so synthetisierten Aprotinin-Homologen können durch Zusatz von organischen mit Wasser mischbaren Lösungsmitteln, wie beispielsweise Glycerin, Butandiol-1,4 und Dimethylformamid gesteigert werden. Bei der enzymatischen Knüpfung der Bindung zwischen den Aminosäuren in Pos. 15 und 16 wird in alkalischer Lösung ein Teil der noch vorhandenen Estergruppen hydrolysiert. Vollständige Hydrolyse erfolgt beim Stehen in 0,001 N - 1,0 N Alkalimetallhydroxidlösungen sowohl bei +4°C wie auch bei Raumtemperatur.

Insbesondere wenn die Knüpfung der Peptidbindung zwischen den Aminosäuren in den Pos. 15 und 16 mit trägergebundenen Enzymen durchgeführt wird, ist es nicht notwendig, die Aprotinin-Homologen vor der Verseifung zu reinigen. Begleitsubstanzen, wie unveränderte Ausgangssubstanz und gegebenenfalls gebildete höhermolekulare Aggregate, vor allem aber die bei der Carbodiimidkondensation gebildeten Acylharnstoffe am Halbcystinrest 14 sind inhibitorisch unwirksam und können durch Filtration abgetrennt werden.

Bei der Carbodiimidkondensation teilweise gebildete Tyrosin-isoharnstoffderivate lassen sich durch Behandeln mit 0,5 M Hydroxylamin bei pH 7,0 und einer Inkubationszeit von 2 - 6 Stunden wieder in underivatisierte Tyrosinreste zurückverwandeln [vgl. K.L. Carraway und D. E. Koshland, Jr., Biochim. Biophys. Acta 160, 272 (1968)]. Beim Ansäuern der Suspension auf pH-Werte von 1,5-3 werden die Aprotinin-Homologen aus ihren Komplexen freigesetzt und treten in das Filtrat über.

Wird die 15-16-Bindung dagegen mit löslichen Enzymen geknüpft, so empfiehlt es sich, die Enzyme in an sich bekannter Weise durch Säurefällung oder Gelchromatographie abzutrennen.

Die Resynthese der Peptidbindung 15/16 im reaktiven Zentrum gelingt auch weitgehend mit Hilfe wasserlöslicher Carbodiimide bei pH-Werten von 3-7, vorzugsweise bei pH 4,5-5 und besonders gut in Gegenwart von Hydroxysuccinimid. Hierzu müssen allerdings die Estergruppen, vorzugsweise die an der neu eingeführten Aminosäure 15 verseift werden, was wie oben beschrieben mit Natronlauge oder wegen ihrer esterolytischen Aktivität mit geeigneten Proteinasen durchgeführt werden kann. Nachteile der peptidchemisch durchgeführten Resynthese sind im Gegensatz zu der enzymatisch katalysierten Resynthese die niedrigere Ausbeute an gewünschtem Homologen und die Bildung unerwünschter Nebenprodukte durch intra- und intermolekulare Kondensationsreaktionen sowie Addition des Carbodiimids an die Carboxylgruppen zu Acylharnstoffen.

Bequem gelingt die Trennung von inhibitorisch wirksamen und inhibitorisch unwirksamen Substanzen auch, wenn man die bei der enzymatischen Kondensation anfallende Reaktionslösung zunächst unter neutralen bzw. alkalischen Bedingungen durch Gelfiltration fraktioniert. Das Aprotinin-Homologe wird nur in den den Komplex enthaltenden Fraktionen eluiert. Nach dem Einstellen des pH-Werten der Lösung auf Werte zwischen pH 1 und 4 dissoziiert der Enzyminhibitorkomplex und Enzym und Inhibitor können durch eine nochmalige Gelfiltration unter sauren Bedingungen getrennt werden.

Bevorzugte Lösungsmittel für diese Filtrationen sind Essigsäure, Ameisensäure oder auch wäßrige Mineralsäuren oder Gemische dieser Lösungsmittel.

Es ist besonders vorteilhaft, für die Isolierung und Reinigung der erfindungsgemäßen Inhibitoren ihre große Affinität zu den Proteinasen auszunutzen. Dies geschieht zweckmäßigerweise entweder nach dem ersten, dem chemischen Kondensationsschritt, oder aber nach der zweiten, enzymatischen Umsetzung durch Fraktionierung unter Verwendung von affinitätschromatographischen Verfahren. Als Affinitätsadsorbentien werden zumeist feste inerte Substanzen (Träger) verwendet, auf deren Oberfläche die betreffenden Enzyme nach an sich bekannten Verfahren fixiert sind. Beim Auftrag und der anschließenden Elution der Reaktionsgemische worden die inhibitorisch unwirksamen Substanzen sofort, inhibitorisch wirksame Substanzen dagegen verzögert eluiert oder ganz zurückgehalten. Die Bindungen zwischen dem trägergebundenen Enzym und den Inhibitoren können dann durch Variation von pH-Wert und/oder der Salzkonzentration des Elutionsmittels aufgehoben werden, so daß schließlich die inhibitorisch aktiven Komponenten der Mischung im Eluat erhalten werden.

Neben der Gelfiltration und der Affinitätschromatographie eignet sich insbesondere die Ionenaustauschchromatographie für die Entsalzung und Fraktionierung der Reaktionsgemische.

Die neuen erfindungsgemäßen Aprotinin-Homologen, die sich vom Aprotinin nur durch den Austausch des Aminosäurerestes in Pos. 15 unterscheiden, sind wertvolle Proteinaseninhibitoren mit veränderten Wirkungen und Wirksamkeiten, die auf die veränderten Hemmspektren zurückzuführen sind.

Diese Änderungen des Hemmspektrums der erfindungsgemäßen Aprotinin-Homologen sind bedingt durch den Austausch von Lys-[15] im aktiven Zentrum des Aprotinin gegen Aminosäuren, deren Seitenketten besser in die Spezifitätstaschen der betreffenden Proteinasen passen. Nach den Ergebnissen der Arbeitsgruppen von Powers und Zimmerman bei Untersuchungen über die Affinität von synthetischen Substraten und Inhibitoren für Granulozytenelastase, Pankreaselastase, Chymotrypsin und Kathepsin G überrascht die gute Hemmwirkung des Leu[15]-Aprotinin und ebenso des Norleucin[15]-Aprotinin für alle diese Enzyme und die relativ große Selektivität des Homologen mit Val in Pos. 15 für die Granulozytenelastase sowie dessen Collagenasehemmung. Außerdem überrascht, daß das Ala[15]-Aprotinin beide Elastasen nur schwach hemmt. Bemerkenswert ist auch die hervorragende antitryptische Wirkung von Gly[15]-Aprotinin und die überraschende Hemmung von Gewebs-Kininogenase (Kallikrein).

Die erfindungsgemäßen Inhibitoren haben gegenüber dem Aprotinin überlegene biologische Eigenschaften. Von besonderem Vorteil sind ihre inhibitorischen Wirkungen auf die Elastasen aus Pankreas und Granulozyten sowie auf Kathepsin G und die Granulozytencollagenase, die neue therapeutische Einsatzmöglichkeiten eröffnen. Pankreas-Elastase spielt eine wichtige Rolle bei der Pankreatitis; Serumelastase bei der Atherosklerose und Granulozytenelastase bei akuten und chronischen Entzündungen mit Bindegewebeschädigung, bei Gefäßwandschädigungen sowie bei nekrotisierenden Erkrankungen und Degeneration von Lungengewebe, z. B. beim Emphysem. Ebenso wichtig ist die Rolle von lysosomalen Enzymen und insbesondere der Granulozytenelastase bei immunologisch bedingten Entzündungsreaktionen, z. B. der rheumatoiden Arthritis.

**Tabelle 1**

Aminosäurezusammensetzung von Aprotinin und einigen Homologen nach Spackman,
Stein und Moore, 1958, Anal. Chem. 30, 1190 [1]

| Amino-säure | Aprotinin (theoret.) | Arg[15]-Aprotinin gef. | theoret. | Gly[15]-Aprotinin gef. | theor. | Val[15]-Aprotinin gef. | theor. |
|---|---|---|---|---|---|---|---|
| Asp | 5 | 5,08 | 5 | 4,98 | 5 | 5,03 | 5 |
| Thr | 3 | 2,93 | 3 | 2,95 | 3 | 2,89 | 3 |
| Ser | 1 | 1,38 | 1 | 1,38 | 1 | 1,04 | 1 |
| Glu | 3 | 2,93 | 3 | 2,93 | 3 | 2,79 | 3 |
| Pro | 4 | 3,76 | 4 | 3,81 | 4 | 3,81 | 4 |
| Gly | 6 | 6,00 | 6 | 7,02 | [7] | 6,00 | 6 |
| Ala | 6 | 5,89 | 6 | 6,00 | 6 | 5,79 | 6 |
| Cys | 6 | 5,13 | 6 | 4,88 | 6 | 5,01 | 6 |
| Val | 1 | 0,62 | 1 | 0,95 | 1 | 1,94 | [2] |
| Met | 1 | 0,65 | 1 | 0,55 | 1 | 0,70 | 1 |
| Ile | 2° | 1,15 | 2 | 1,18 | 2 | 1,36 | 2 |
| Leu | 2 | 1,93 | 2 | 1,89 | 2 | 2,01 | 2 |
| Tyr | 4 | 3,66 | 4 | 3,37 | 4 | 3,58 | 4 |
| Phe | 4 | 4,04 | 4 | 3,68 | 4 | 3,84 | 4 |
| Lys | 4 | 2,58 | 3 | 3,02 | 3 | 2,95 | 3 |
| Arg | 6 | 6,80 | [7] | 5,74 | 6 | 5,96 | 6 |

[1] Die Molverhältnisse sind bezogen auf Gly = 6,0 bzw. Ala = 6,0 beim Gly[15]-Aprotinin
°) Die Isoleucinwerte werden wegen einer Ile-Ile-Bindung im Molekül nach 18 Stunden Hydrolyse zu niedrig gefunden.

Die erfindungsgemäßen Inhibitoren lassen sich durch chemische, physikalisch-chemische, biochemische sowie biologische Eigenschaften charakterisieren. Es würden folgende Kriterien herangezogen:

6

### 1. Aminosäurezusammensetzung

Die Aminosäurezusammensetzung wurde nach S. Moore, D. H. Spackman, W.H. Stein [Anal. Chem. 30, 1185 (1958)] bestimmt. Für einige der erfindungsgemäßen Aprotinin-Homologe sind in Tabelle 1 beispielhaft die Werte der Aminosäureanalysen zusammengestellt.

### 2. Hochdruckflüssigkeitschromatographie

Die HPLC wurde mit dem Hewlett-Packard (HP) Modell 1084B durchgeführt unter Einsatz einer Bio-Sil TSK IEX 530 CM-Säule 4 x 300 mm (Bio-Rad Labs, Richmond, USA). Die Fließgeschwindigkeit betrug 1 ml/min unter Verwendung eines linearen Gradienten aus 0,15 M Natriumsulfat und 0,6 M Natriumsulfat enthaltenden pH 7,0-Puffern (Fixanal 38746 Riedel de Haen) bei einer Säulentemperatur von 40°C und einem Druck von ca. 50 bar. Je Lauf wurden 20 µl einer Lösung von 1 mg Aprotinin-Derivat in 1 ml Wasser aufgegeben, die Detektion erfolgte bei 215 nm und bei 280 nm. Als interner Standard wurde Aprotinin verwendet; die angegebenen Retentionszeiten beziehen sich auf Aprotinin.

### 3. Elektrophoresen

Die Elektrophoresen wurden unter den von Jering und H. Tschesche [Eur. J. Biochem. 61, 443 (1967)] angegebenen Bedingungen durchgeführt. Anstelle von 10 % Acrylamid wurde jedoch 7 % Acrylamid verwendet. Die elektrophoretischen Beweglichkeiten der Aprotinin-Homologen wurden bezogen auf die von Aprotinin als Standard.

### 4. Proteasen-Inhibitionsspektrum

#### a) Elastase-Inhibition

#### α) Pankreas-Elastase-Inhibition

Für die Hemmversuche mit den erfindungsgemäßen Aprotinin-Homologen wurde kristallisierte Pankreas-Elastase (Schwein) der Fa. Nutritional Biochemicals Corp. verwendet. Als Substrat wurde Succinyl-L-alanyl-L-alanyl-L-alanin-p-nitroanilid [J. Bieth, et al, Biochem. Med. 11, 350 (1974)] eingesetzt. Die Spaltung wurde durch kontinuierliche Messung der Extinktion des freigesetzten p-Nitroanilins bei 405 nm bestimmt. Um maximale Komplexbildung sicherzustellen, wurden Enzym und Inhibitor vor der Zugabe des Substrats für 15 Min. vorinkubiert. In Tabelle 2 sind semiquantitative Angaben über die Inhibition des Enzyms für einige der neuen Inhibitoren zusammengestellt.

#### β) Granulozyten-Elastase-Inhibition

Das für die Hemmteste verwendete Isoenzymgemisch wurde nach K. Ohlsson und I. Olsson [Europ. J. Biochem, 42, 519 (1974)] aus Humangranulozyten gewonnen. Als Substrat ist besonders Succinyl-L-alanyl-L-alanyl-L-valin-p-nitroanilid [H. R. Wenzel et al., Hoppe Seyler's Z. Physiol. Chem. 361, 1413 (1980)] geeignet. Angaben über die Hemmung von Granulozytenelastase durch einige erfindungsgemäße Aprotinin-Homologen sind in Tabelle 2 aufgenommen.

#### b) Chymotrypsin-Inhibition

Die Aktivität von Chymotrypsin wurde nach W. Nagel, et al, Hoppe-Seylers Z. Physiol. Chem. 340, 1 (1965) photometrisch mit Succinyl-L-phenylalanin-p-nitroanilid als Substrat bestimmt und die Hydrolyse durch kontinuierliche Messung der Extinktion des freigesetzten p-Nitroanilins bei 405 nm bestimmt. Vor der Zugabe des Substrates wurden Enzym und Inhibitor im Testpuffer 15 Minuten vorinkubiert. In Tabelle 2 sind Angaben zur Chymotrypsinhemmung für einige der erfindungsgemäßen Aprotinin-Homologen enthalten.

#### c) Kathepsin G-Inhibition

Die Aktivität von Kathepsin G wurde mit Succinyl-L-phenylalanin-β-naphthylester als Substrat durch kontinuierliche spektroskopische Bestimmung des bei der enzymatischen Spaltung freigesetzten β-Naphthols bei 328,5 nm bestimmt. Die Enzymreaktion wird in einem 0,25 M pH 7,2 Trishydroxymethylaminomethan-Salzsäurepuffer durchgeführt, der 0,05 % Brij 35 enthält und 0,005 M an Magnesiumchlorid ist. Nach 15 Min. Vorinkubation von Enzym und Inhibitor in 2,5 ml Testpuffer werden 0,025 ml einer Stammlösung von 39,4 mg Substrat in 1 ml Dimethylsulfoxid zugesetzt und der Extinktionsanstieg bei 328,5 nm bestimmt. Von dieser Extinktion wird der durch Spontanhydrolyse bedingte Extinktionsanstieg abgezogen. In Tabelle 2 sind die Ergebnisse für einige der erfindungsgemäßen Aprotinin-Homologen enthalten.

#### d) Trypsin-Inhibition

Die Trypsinaktivität wurde nach H. Fritz et al, [in Methoden der enzymatischen Analyse, Herausgeber H. W. Bergmeyer, 2. Auflage, Band 1, 1011 (1970)] mit Benzoyl-L-arginin-p-nitroanilid als Substrat bestimmt. Das freigesetzte p-Nitro-anilin wurde spektrophotometrisch bei 405 nm gemessen. Enzym und Inhibitor wurden vor der Zugabe des Substrats 15 Min. vorinkubiert. In Tabelle 2 finden sich Angaben zur Hemmbarkeit von Trypsin durch einige der erfindungsgemäßen Aprotinin-Homologen.

#### e) Pankreas-Kininogenase-Inhibition Pankreas-Kallikrein

Als Testenzym wurde Pankreas-Kallikrein (Schwein) verwendet. Die Bestimmung der Enzymaktivität wurde mit dem Substrat D-Valyl-L-leucyl-L-arginin-p-nitroanilid (A. B. Kabi) nach T. Dietl et al. [Hoppe-Seyler's Z. Physiol. Chem. 360, 67 (1979)] durchgeführt. Vor der Zugabe des Substrats wurden Enzym und Inhibitor 15 Min. vorinkubiert. Die Hydrolyse wurde spektrophotometrisch durch Bestimmung des freigesetzten p-Nitroanilins bei 405 nm verfolgt. Angaben zur Hemmbarkeit von Schweinepankreas-Kallkrein finden sich in Tabelle 2.

#### f) Collagenase-Inhibition

Die Granulozytencollagenase wurde nach H. W. Macartney und H. Tschesche [FEBS Letters 119, 327 (1980)] aus Humanleukozyten isoliert und die Bestimmung der Enzymaktivität nach dem in der obigen Arbeit angegebenen Verfahren durchgeführt. In Tabelle 2 ist die Hemmbarkeit der Granulozytencollagenase durch einige der erfindungsgemäßen Aprotinin-Homologen qualitativ angegeben.

#### g) Faktor Xa-Inhibitoren

Humaner Faktor X wurde von der Fa. Boehringer erworben -5 E in 0,5 ml -.
Als Substrat wurde Benzoyl-L-isoleucyl-L-glutamyl-glycyl-L-arginin-p-nitroanilid (S-2222 der Fa. A. B. Kabi) verwendet. Die Bestimmung der Enzymaktivität wurde anhand der p-Nitroanilinfreisetzung nach 10 Min. Vorinkubation von Enzym und Inhibitor durchgeführt. Tabelle 2 enthält Angaben zur Wirksamkeit einiger der erfindungsgemäßen Aprotinin-Homologen.

#### h) Plasmin-Inhibition

Die plasmininhibitorische Wirkung einiger der erfindungsgemäßen Aprotinin-Homologen ist qualitativ aus Tabelle 2 ersichtlich. Die Bestimmungen wurden durchgeführt mit Humanplasmin unter Verwendung des Substrates D-Valyl-L-leucyl-L-lysin-p-nitroanilid (S-2251; Fa. A. B. Kabi) nach einer 10 minütigen Vorinkubation von Enzym und Inhibitor in einem 0,1 M Tris-(hydroxymethyl)-aminmethan-Salzsäurepuffer, pH 7,4, der 0,05 M Natriumchlorid enthielt.

**Tabelle 2**

Semiquantitative Angaben zur Hemmbarkeit* einiger wichtiger Proteinasen durch einige der erfindungsgemäßen Aprotinin-Homologen.

| Aminosärerest in Position 15 des Inhibitiors | Chymotrypsin | Kathepsin | G Collagenase aus Granulozyten | Elastase Granulozyten | Pankreas | Kallikrein | Trypsin | Plasmin | Faktor Xa |
|---|---|---|---|---|---|---|---|---|---|
| Lys (Aprotinin) | ++ | (+) | - | (+) | - | +++ | +++ | +++ | - |
| Gly | ++ | n.b. | n.b. | - | - | +++ | +++ | n.b. | n.b. |
| Ala | + | n.b. | ++ | + | + | + | + | n.b. | n.b. |
| Met | +++ | ++ | n.b. | + | + | + | + | n.b. | n.b. |
| Val | + | n.b. | ++ | +++ | + | + | + | n.b. | n.b. |
| Leu | +++ | ++ | n.b. | +++ | +++ | + | + | n.b. | n.b. |
| Arg | ++ | (+) | n.b. | (+) | - | +++ | +++ | +++ | + |

*: - keine Hemmung; + schwache Hemmung; ++ starke Hemmung; +++ sehr starke Hemmung; n.b. = nicht bestimmt; (+) Affinität zum Enzym vorhanden

**0 132 732**

Die erfindungsgemäßen Aprotinin-Homologen sind in Modellen der akuten Entzündungsreaktion dem Aprotinin überlegen, da mit ihnen nicht nur in deutlich geringeren Dosierungen die gleiche Wirkung wie mit Aprotinin erzielt wird, sondern die Entzündungsreaktionen auch dann signifikant gehemmt wird, wenn sie mehrere Stunden nach Setzen der Entzündungsnoxe verabreicht werden. Eine solche therapeutische Wirkung ist mit dem Aprotinin im Kaolin- und Aerosilmodell bei einmaliger Gabe nicht zu erreichen.

<u>Versuchsanordnung zum Nachweis entzündungshemmender Wirkung bei der Ratte</u>

a) <u>Kaolin-induzierte Entzündungsreaktion</u>

Die Entzündungsreaktion wurde durch intraplantare Injektion von 0,1 ml einer 10 %-igen Kaolinsuspension in eine Hinterpfote von 130-160 g schweren Wistar-Ratten induziert. Die für die Behandlung der Entzündungsreaktion verwendeten erfindungsgemäßen Aprotinin-Homologen wurden in 0,9 %-iger Natriumchloridlösung in einer Konzentration von 10-20 mg/ml gelöst. Die Behandlung der Versuchstiere erfolgte durch intraperitoneale, intramuskuläre, subkutane oder intravenöse Injektion von 0,5-1,0 ml Lösung der Inhibitoren und zum Vergleich Aprotinin entweder prophylaktisch, d.h. <u>vor</u> Setzen der Entzündungsnoxe, oder therapeutisch, d.h. <u>nach</u> Setzen der Entzündungsnoxe. Die Schwellung der entzündeten Pfote, die ein Maß für die Schwere der Entzündungsreaktion ist, wurde mit dem Antiphlogmeter nach Kemper [F. Kemper und G. Ameln, Z. ges. exp. Med. <u>131</u>, 407-411 (1959)] zeitlich verfolgt.

Zur Ermittlung der Dosis-Wirkungsbeziehungen wurde der 4 Stunden nach Setzen der Entzündungsnoxe gemessene Wert verwendet.

b) <u>Aerosil-induzierte Entzündungsreaktion</u>

Die Entzündungsreaktion wurde durch intraplantare Injektion von 0,1 ml einer 2 %-igen Aerosilsuspension in eine Hinterpfote von 130-160 g schweren Wistar-Ratten induziert. Die für die Behandlung der Entzündungsreaktion verwendeten erfindungsgemäßen Aprotinin-Homologen bzw. Aprotinin wurden in 0,9 %-iger Natriumchloridlösung in einer Konzentration von 10-20 mg/ml gelöst. Die Behandlung der Versuchstiere erfolgt durch intraperitoneale, subkutane oder intravenöse Injektion von 0,5-1,0 ml Lösung der Inhibitoren und zum Vergleich Aprotinin 15 h nach Setzen der Entzündungsnoxe. Die Schwellung der entzündeten Pfote, die ein Maß für die Schwere der Entzündungsreaktion ist, wurde mit dem Antiphlogmeter nach Kemper zeitlich verfolgt. Zur Ermittlung der Dosis-Wirkungsbeziehungen wurde der 21-Stundenwert nach Entzündungsinduktion (= 6 Stunden nach Injektion der erfindungsgemäßen Aprotinin-Homologen bzw. Aprotinin ermittelt), verwendet.

Das Ergebnis der Therapieversuche mit den neuen Inhibitoren nach den Beispielen zeigt die Wirksamkeit der verwendeten Aprotinin-Homologen in diesen experimentellen Modell, in dem Aprotinin in gleicher Dosierung die Entzündungsreaktion nicht hemmt.

Die neuen Inhibitoren können aufgrund ihrer biologischen Wirksamkeit insbesondere zur Behandlung folgender Krankheiten bzw. Krankheitserscheinungen eingesetzt werden:

1. Verschiedene Formen des Schocks, insbesondere Schocklunge und Endotoxinschock, posttraumatische und postoperative Komplikationen,
2. Störungen der Blutgerinnung,
3. Akute und chronische Entzündungsreaktionen, insbesondere zur Therapie und Prophylaxe von Organschädigungen, wie beispielsweise Pankreatitis und strahleninduzierte Enteritis, immunkomplexbedingte Entzündungsreaktionen, wie Immunvasculitis, Glomerulonephritis und Arthritiden; Kollagenosen, insbesondere rheumatoide Arthritis,
4. durch stoffwechselbedingte Ablagerungen verursachte Arthritiden (z.B. Gicht),
5. Degeneration der elastischen Bestandteile der Bindegewebsteile von Organen, wie bei der Atherosklerose, oder dem Lungenemphysem
6. Strahleninduzierte Enteritis.

Die neuen Wirkstoffe können in bekannter Weise (analog dem Aprotinin) in übliche Formulierungen übergeführt werden.

Folgende Formulierungen sind dabei bevorzugt zu nennen:

1. Lösungen für parenterale Anwendung zur intravenösen, intramuskulären, subkutanen Injektion bzw. zur intraartikulären und intratumoralen Injektion.
2. Lösungen für intravenöse Dauerinfusion,
3. Lösungen zur Anwendung als Aerosole zur Inhalation,
4. Lösungen, Emulsionen, Salben, Pasten, Cremes, Lotions, Puder zur äußerlichen lokalen Anwendung.
5. Kombination verschiedener Hemmstoffe, deren Wirkungsspektrum sich gegenseitig ergänzt.

0 132 732

Die Konzentrationen der neuen Wirkstoffe in entsprechenden Formulierungen bewegen sich dabei in den Grenzen 0,01 bis 100 mg/ml Lösung, vorzugsweise zwischen 0,1 bis 10 mg/ml Lösung.

Die neuen Wirkstoffe können in üblicher Weise angewendet werden, insbesondere sind folgende Anwendungsmethoden als bevorzugt zu nennen:

a)           parenteral: intravenös, intramuskulär, subkutan, intraartikulär, intratumoral
b)           lokal: z. B. intranasal
c)           oral.

Als Dosierungsbereich kann für die erfindungsgemäßen Wirkstoffe angegeben werden:

0,1 - 20 mg Wirkstoff/kg Körpergewicht, vorzugsweise 1 bis 10 mg Wirkstoff/kg Körpergewicht, die Dosierung ist dabei vor allem abhängig von der zu behandelnden Spezies sowie von der Applikationsart.

Die erfindungsgemäßen Wirkstoffe können bei Mensch und Tier eingesetzt werden.

**Beispiel 1**

Des-Lys-15-Aprotinin*-pentamethylester

a) Aprotinin*-hexamethylester

160 mg Aprotinin* (~ 25 µM) wurden gelöst in 45 ml Methanol, das 0,1 M an Chlorwasserstoff war und diese Lösung bei Raumtemperatur (20°C) gehalten. Eine evtl. auftretende Fällung wurde durch Zugabe von weiterem Methanol gelöst und die Veresterung durch HPLC und Chromatographie an CM Sephadex C-25 kontrolliert. Nach ca 150 h wurde das Lösungsmittel im Vakuum abdestilliert und der Rückstand mit 15 ml Methanol übergossen, das sofort abdestilliert wurde.

Nach mehrmaliger Wiederholung des Löse-Abdampfvorgangs wurde der Rückstand in 20 ml Wasser gelöst. Beim Lyophilieren wurden 150 mg Aprotinin*-hexamethylester als farblose Substanz erhalten. Relative elektrophoretische Beweglichkeit: 1,61 (pH 5,0-Puffer); relative Retentionszeit in der HPLC: 2,04 (für Aprotinin* 0,59). Die Substanz wurde in 10 ml 0,05 M Kaliumphosphatpuffer pH 4,5 gelöst und diese Lösung aufgetragen auf eine mit dem Lösungspuffer äquilibrierte CM-Sepharose CL-4B Fast Flow gefüllte Säule (2,5-45 cm). Die Säule wurde entwickelt mit einem Gradienten aus Auftragspuffer und 0,8 M Natriumchlorid enthaltenden Startpuffer. Der Aprotinin*-hexamethylester wird bei einer Natriumchloridkonzentration von 0,6-0,7 M von der Säule eluiert. Die die Substanz enthaltenden Eluate wurden gepoolt und durch Ultrafiltration über eine Amicon UM-02 Membrane entsalzt und mit 0,1 M Essigsäure nachgewaschen. Bei der Lyophilisation des Retentats wurden 125 mg farblose Substanz erhalten.

b) Di-Cysteinyl-14,38-Aprotinin*-hexamethylester

104 mg Aprotinin*-hexamethylester (~ 16 µMol) wurden gelöst in 12 ml 0,1 M sauerstoff-freiem Phosphatpuffer pH 5.8. Zu dieser Lösung fügte man 154 mg Dithiothreitol (mMol) zu. Nach 6,5-stündigem Stehen der Reaktionslösung unter einer Stickstoffatmosphäre wurde mit Essigsäure ein pH-Wert von 3 eingestellt. Man filtrierte das Gemisch zum Abtrennen des Dithiothreitol über Sephadex G-25 (Säulendimensionen: 2,5 x 100 cm) und lyophilisierte die proteinhaltigen Filtrate. Es wurden 95 mg farblose Substanz erhalten.

c) Di-Cysteinyl-14,38-Aprotinin*-pentamethylester

90 mg (~ 14 µMol) nach 1b) erhaltene Substanz wurden in 10 ml 0,1 M Phosphatpuffer, pH 6,75, gelöst und versetzt mit 1 ml einer Lösung von 25 mg an den Tryptophanresten formyliertem Trypsin in 2 ml 8 M Harnstofflösung, pH 8,0.

Man hielt die Reaktionslösung 14 h bei 22°C. Dann wurde mit Salzsäure bis pH 2,0 versetzt und die Lösung über eine Sephadex G-50-Säule (2,5 x 120 cm) filtriert mit 0,1 M Essigsäure-Salzsäure, pH 2,0 als Elutionsmittel, um Enzym und Aprotinin*-Derivat zu trennen. Bei der Gefriertrocknung der das Aprotinin*-Derivat enthaltenden Fraktionen wurden 75 mg farblose Substanz erhalten.

d) Aprotinin*-pentamethylester und Des-Lysin-15-Aprotinin*-pentamethylester

Durch eine Lösung von 70 mg nach 1c) erhaltener Substanz in 10 ml 0,1 M Phosphatpuffer, pH 6,25 ließ man bei 22°C einen langsamen Luftstrom perlen, bis zum negativen Ausfall des Ellman-Testes.

Dann setzte man der Reaktionslösung 50 µl einer Suspension von Carboxypeptidase B zu und hielt das Gemisch 1 h bei 22°C. Nach der Zugabe von 0,75 ml Eisessig wurde die Reaktionslösung zum Abtrennen der Carboxypeptidase B und vom abgespaltenen Lysin über eine Sephadex G-50-Säule (2,5 x 125 cm) filtriert, unter Benutzung von 0,1 M Essigsäure als Elutionsmittel. Beim Gefriertrocknen der den Des-Lysin-15-Aprotinin*-pentamethylester enthaltenden Fraktionen wurden 50 mg farblose Substanz erhalten.

**Beispiel 2**

Des-Lysin-15-Aprotinin*-pentamethylester

a) Aprotinin*-pentamethylester

100 mg nach Beispiel 1a) erhaltener Aprotinin*-hexamethylester werden in 48 ml 0,1 M Natriumacetatpuffer, pH 3,5, gelöst. Nach Zugabe von 50 ml Dioxan versetzte man mit 2 ml einer Lösung von 30 mg Trypsin (TPCK-behandelt) / ml $10^{-4}$ N Salzsäure und hielt die Lösung bis zum Ende der Umsetzung (HPLC-Kontrolle) ca. 2 Stunden bei Raumtemperatur. Dann wurde die Reaktionslösung mit 1 ml Eisessig versetzt und im Vakuum auf ein Volumen von 10 ml eingeengt. Nach dem Einstellen von pH 2,0 mit N Salzsäure wurde die Lösung mit 0,1 M Essigsäure-Salzsäure, pH 2,0, als Elutionsmittel über eine Sephadex G-50-Säule (2 x 120 cm) filtriert. Die den Aprotinin*-pentamethylester enthaltenden Eluate wurden nach Zugabe von N Natriumhydroxidlösung bis pH 4,5 im Vakuum auf ein Volumen von 10 ml eingeengt.

b) Des-Lys-Aprotinin*-pentamethylester

Nach Zugabe von 0,1 N Natriumhydroxidlösung bis pH 6,2 zu dem nach a) erhaltenen Kondensat wurden 50 µl einer Suspension von Carboxypeptidase B in die Lösung eingetragen. Man hielt die Lösung 30 Min bei Raumtemperatur und fügte dann 0,5 ml Eisessig hinzu. Zum Abtrennen der Carboxypeptidase filtrierte man die Lösung mit 0,05 M Essigsäure, deren pH-Wert mit Salzsäure auf 2 eingestellt war, über eine Sephadex G-50-Säule (2 x 120 cm) und engte die das Aprotininderivat enthaltenden Eluate nach Zugabe von Natriumhydroxidlösung bis pH 4,0 im Vakuum auf 10 ml ein. Die Entsalzung dieser Lösung erfolgte durch Filtration über eine Bio-Gel P-2-Säule (2 x 100 cm). Beim Gefriertrocknen der entsprechenden Eluate wurden 85 mg Aprotinin*-pentamethylester erhalten.

**Beispiel 3**

L-Valin-15-Aprotinin

a) L-Valin-15-Aprotinin*-hexamethylester

Zu einer Lösung von 13 mg (2µMol) nach Beispiel 2 erhaltenem Des-Lysin-15-Aprotinin*-pentamethylester und 167 mg L-Valinmethylester-hydrochlorid (1 mMol) in 7,5 ml Wasser fügte man bei 20°C 96 mg N-Ethyl-N'-(3-dimethylaminopropyl)-carbo-diimid-hydrochlorid (500 µMol) zu. Durch Zugabe von 0,1 N Salzsäure hielt man den pH-Wert der Reaktionslösung mit Hilfe einer Autotitrators konstant bei 4,75 Nach 2 h wurden die im Reaktionsgemisch enthaltenen niedrigmolekularen Substanzen durch Gelfiltration des Ansatzes über eine Sephadex G-25-Säule (1,5 x 100 cm) mit 0,1 M Essigsäure als Elutionsmittel abgetrennt. Beim Gefriertrocknen der proteinenthaltenden Elaute wurde in quantitativer Ausbeute eine farblose Substanz erhalten.

b) L-Valin-15-Aprotinin-pentamethylester

Das nach 3a) erhaltene Lyophilisat wurde in 15 ml 0,01 M Tris-(hydroxymethyl)-aminomethan-Salzäurepuffer, pH 8,5 24 h mit 12,5 ml Trypsin-Sepharose 4 B - Beladung: 5 mg Trypsin pro ml Gel - unter gelindem Schütteln inkubiert. Dann wurde der pH-Wert des Gemisches mit 0,1 N Salzsäure auf 1,8 eingestellt und das Affinitätsadsorbens durch Filtration abgetrennt und mit insgesamt 20 ml 0,1 M Essigsäure-Salzsäure, pH 2,0 ausgewaschen. Filtrat und Waschwasser wurden vereinigt und die Lösung nach Einstellung eines pH-Wertes von 4 mit N Natriumhydroxidlösung im Vakuum auf ein Volumen von 10 ml eingeengt. Das Konzentrat wurde durch Filtration über eine Sephadex G-25-Säule (1,5 x 100 cm) entsalzt. Bei der Lyophilisation der proteinenthaltenden Eluate wurden 13 mg farblose Substanz erhalten.

c) L-Valin-15-Aprotinin

Die nach Beispiel 3b) erhaltene Substanz wurde in 10 ml 0,001 N Natriumhydroxidlösung gelöst. Nach 12-stündigem Stehen bei 21°C wurde die Lösung mit 0,01 N Salzsäure neutralisiert und nach Zugabe von 550 mg Hydroxylammoniumchlorid 6 Stunden bei Raumtemperatur gehalten. Man trug die Lösung auf eine mit 0,01 M Boratpuffer, pH 8,6 äquilibrierte CM-Sephadex C-25-Säule (2 x 40 cm) auf. Die Säule wurde mit einem linearen Gradienten aus dem Äquilibrierpuffer und dem 0,4 M Natriumchlorid enthaltenden Äquilibrierpuffer eluiert. Die Granulozytenelastase-hemmenden Eluate - Elution bei einer Natriumchloridkonzentration von 0,25 M - wurden vereinigt und die Lösung nach dem Einengen durch Gelfiltration über eine Bio-Gel P-2-Säule (1,5 x 100 cm) mit 0,1 M Essigsäure als Eluans entsalzt. Nach Lyophilisation wurden 5,7 mg Valin-15-Aprotinin erhalten. (45 % bezogen auf den eingesetzten Des-Lysin-15-Aprotinin*-pentamethylester); Relative elektrophoretische Beweglichkeit: 0,54.

**Beispiel 4**

L-Leucin-15-Aprotinin

Analog wie bei der Synthese von Valin-15-Aprotinin wurden 13 mg Des-Lysin-15-Aprotinin*-pentamethylester (2 μ Mol) und 92 mg L-Leucinmethylester-hydrochlorid (500 μ Mol) mit Hilfe von 58 mg N-Ethyl-N'-(3-dimethylaminopropyl)-carbodiimid-hydrochlorid (300 μMol) kondensiert.

Die Reaktionslösung wurde mit 50 ml 0,05 M Tris-(hydroxymethyl)-aminomethan-Salzsäurepuffer, pH 8,5 verdünnt und 20 ml α-Chymotrypsin-Sepharose CL-4B - Beladung: 7 mg Enzym je ml Gel - in die Mischung eingetragen. Nach 24 h gelindem Schütteln wurde von dem Affinitätsträger abfiltriert und das Gel gut mit dem obigen Puffer nachgewaschen und schließlich mit 250 ml Wasser.

Man suspendierte die α-Chymotrypsin-Sepharose CL-4B in 50 ml 0,1 M Essigsäure und versetzte bis zum Erreichen von pH 1,8 mit 0,1 N Salzsäure. Nach 20 Min. Stehen wurde erneut abgesaugt und der Träger mit insgesamt 100 ml 0,1 M Essigsäure in mehreren Anteilen gewaschen.

Die vereinigten Filtrate wurden nach dem Einstellen auf pH 3,0 mit N Natriumhydroxidlösung im Vakuum auf ein Volumen von ~ 5 ml eingeengt und das Konzentrat mit 0,1 N Natriumhydroxidlösung neutralisiert. Nach Zugabe von 0,5 ml 0,05 N Natriumhydroxidlösung ließ man diese Mischung über Nacht bei 20°C stehen und neutralisierte nach 14 h mit 0,1 M Essigsäure. Nach Zugabe von 300 mg Hydroxylammoniumchlorid hielt man die Lösung 4 Stunden bei Raumtemperatur.

Die Lösung wurde durch Gelfiltration über eine Sephadex G-25-Säule (1,5 x 100 cm) entsalzt und die proteinenthaltenden Eluate vereinigt und gefriergetrocknet. Es wurden 7,4 mg einer farblosen Substanz (~ 58 %) erhalten; Relative elektrophoretische Beweglichkeit: 0,58.

**Beispiel 5**

Glycin-15-Aprotinin

a) Glycin-15-Aprotinin*-hexamethylester

13 mg Des-Lysin-15-Aprotinin*-pentamethylester (2 μMol) wurden unter Verwendung von 125,5 mg Glycinmethylesterhydrochlorid (1 mMol), wie bei Beispiel 3a) beschrieben, mit Hilfe von 94 mg (750 μMol) N-Ethyl-N'-(3-dimethylaminopropyl)-carbodiimid-hydrochlorid kondensiert und der Glycin-15-Aprotinin*-hexamethylester nach Gelfiltration, wie dort beschrieben, in quantitativer Ausbeute isoliert.

b) Glycin-15-Aprotinin

Zu der Lösung der nach 5a) erhaltenen Substanz in 10 ml 0,05 M Phosphatpuffer, pH 3,7 fügte man 0,25 ml einer 0,1 M Calciumchloridlösung, deren pH-Wert mit 1 N Natriumhydroxidlösung auf 6,0 eingestellt worden war und anschließend 60 mg Rindertrypsin (80 %-ig) (2 μMol). Nach 30 min. Stehen bei 20°C wurde der pH-Wert der Lösung mit 1 N Natriumhydroxidlösung auf 8,0 gestellt und zur Trennung von Trypsin und Trypsin-Glycin-15-Aprotinin-pentamethylester-Komplex einerseits und inaktiven Des-Lysin-15-Aprotinin*-Derivaten andererseits über eine Sephadex G-50 Säule (2 x 100 cm) filtriert unter Verwendung von 0,05 M Tris-(hydroxymethyl)-aminomethan-Salzsäurepuffer pH 7,5 als Elutionsmittel. Die dem Komplex bzw. Trypsin entsprechenden Eluate wurden vereinigt und mit 2,5 ml 0,1 N Natriumhydroxidlösung versetzt. Nach 14-stündigem Stehen der Reaktionslösung bei 22°C neutralisierte man mit 1 M Essigsäure und konzentrierte im Vakuum auf ein Volumen von 10 ml. Zu dem Konzentrat fügte man 550 mg Hydroxylammoniumchlorid und beließ die Lösung 4 Stunden bei Raumtemperatur. Der PH-Wert der Lösung wurde mit konz. Salzsäure auf 2 eingestellt und das Gemisch ohne vorheriges Abtrennen des gebildeten Niederschlages auf eine Sephadex G-50-Säule (2 x 100 cm) aufgetragen. Die Säule wurde mit 0,1 M Essigsäure-Salzsäure, pH 2, entwickelt. Aus den das Glycin-15-Aprotinin enthaltenden Elauten wurden nach dem Einengen und Entsalzen durch Gelfiltration über eine Bio-Gel P-2-Säule (1,5 x 100 cm) durch Gefriertrocknen 8,3 mg (64 %) farblose Substanz erhalten.

**Beispiel 6**

L-Arginin-15-Aprotinin

a) L-Arginin-15-Aprotinin-oligomethylester

13 mg Des-Lysin-15-Aprotinin*-pentamethylester (2 μMol) wurden mit 130,5 mg L-Argininmethylester-dihydrochlorid (500 μMol) in Gegenwart von 57 mg N-Ethyl-N'-(3-dimethylaminopropyl)-carbodiimid-hydrochlorid umgesetzt, wie bei Beispiel 3a) beschrieben.

Durch Zugabe von 1 N Natriumhydroxidlösung stellte man den pH-Wert der Lösung anschließend auf 7,5 ein und setzte 1 ml einer 0,1 M Calciumchloridlösung und 80 ml 0,05 M Tris-(hydroxymethyl)-aminomethan-Salzsäurepuffer, pH 7,5 zu. Nach dem Nachstellen des pH-Wertes wurden 20 ml Trypsin-Sepharose CL-4B - Enzymgehalt: 5 mg je ml Gel - in die Lösung eingetragen und die Suspension 15 h gelinde geschüttelt. Dann wurde vom Affinitätsträger abfiltriert und das Gel mit 50 ml des obigen Puffers und schließlich mit 100 ml Wasser gewaschen. Man suspendierte die Trypsin-Sepharose in 100 ml 0,1 M Essigsäure-Salzsäure, pH 1,8. Nach dem Nachstellen des pH-Wertes wurde 20 Min. sachte geschüttelt und erneut filtriert. Das Affinitätsgel wurde gut mit 100 ml 0,1 M Essigsäure-Salzsäure, pH 1,8 in mehreren Portionen gewaschen.

Die vereinigten Filtrate wurden nach dem Einstellen eines pH-Wertes von 4,5 mit N Natriumhydridlösung im Vakuum auf ein Volumen von ca. 10 ml konzentriert, nachdem man zuvor mit 5 ml N Natriumhydroxidlösung versetzt hatte. Das Konzentrat wurde über eine Sephadex G-25-Säule (2 x 100 cm) filtriert mit 0,1 M Essigsäure als Elutionsmittel. Aus den Protein enthaltenden Eluaten wurden durch Lyophilisation 9,6 mg (76 %) Arginin-15-Aprotinin-oligomethylester erhalten.

b) L-Arginin-15-Aprotinin

Die nach 6a) erhaltene Substanz wurde in 10 ml 0,001 M Natriumhydroxidlösung gelöst und diese Lösung 24 h bei 20°C gehalten. Dann wurde mit 0,1 M Salzsäure neutralisiert und 500 mg Hydroxylammoniumchlorid zu der Lösung hinzugefügt. Nach 3 stündigem Stehen der Mischung bei Raumtemperatur wurde diese Lösung zur Entsalzung unter Verwendung von 0,1 M Essigsäure als Elutionsmittel über eine Sephadex G-25-Säule (2 x 100 cm) filtriert. Die proteinenthaltenden Eluate wurden nach dem Einengen gefriergetrocknet. Man erhielt 8,2 mg (86 % bzw. 65 % bezogen auf den Des-Lysin-15-Aprotinin*-pentamethylester) farblose Substanz mit einer relativen elektrophoretischen Beweglichkeit von 1,05 und einer relativen Retentionszeit von 1,13 in der HPLC.

**Beispiel 7**

L-Norleucin-15-Aprotinin

Die bei der Umsetzung von 13 mg Des-Lysin-15-Aprotinin-*-pentamethylester (2 μMol) und 55 mg L-Norleucinmethylester-hydrochlorid (300 μMol) mit 83 mg N-Cyclohexyl-N'-[2-(4-morpholinyl)-ethyl]-carbodiimid-methyl-p-toluolsulfonat (200 μMol) analog Beispiel 3a) erhaltene Substanz wurde zur Synthese der Peptidbindung zwischen Norleucin 15 und Alanin 16 in 50 ml 0,1 M Tris-(hydroxymethyl)-aminomethan-Salzsäurepuffer, pH 8,0, mit 20 ml Chymotrypsin-Sepharose CL-4B - Beladung: 7 mg Enzym je ml Gel - 6 Stdn. inkubiert.

Das Gel wurde mit dem obigen Inkubationspuffer und schließlich mit 100 ml Wasser gewaschen. Man suspendierte anschließend in 50 ml 0,1 M Essigsäure-Salzsäure, pH 1,8 und trennte Gel und Flüssigkeit durch Filtration. Das Affinitätsgel wurde mit 100 ml 0,05 M Essigsäure-Salzsäure nachgewaschen und die vereinigten Filtrate nach der Zugabe von konz. Ammoniumhydroxidlösung bis pH 4,0 im Vakuum auf ein Volumen von ~ 10 ml eingeengt. Das Konzentrat wurde neutralisiert und nach Zugabe von 550 mg Hydroxylammoniumchlorid 3 Stunden bei Raumtemperatur belassen. Zur Entsalzung wurde die Lösung über Bio-Gel P-2-Säule (2 x 100 cm) filtriert mit 0,1 M Essigsäure als Elutionsmittel. Die proteinenthaltenden Eluate wurden konzentriert und lyophilisiert. Man erhielt 9,2 mg (71 %) farblose Substanz.

**Beispiel 8**

Norvalin-15-Aprotinin

Das bei der Kondensation von 13 mg Des-Lysin-15-Aprotinin*-pentamethylester (2 μMol) und 67 mg L-Norvalinmethylester-hydrochlorid (400 μMol) in Gegenwart von 58 mg N-Ethyl-N'-(3-dimethylaminopropyl)-carbodiimid-hydrochlorid (300 µMol) erhaltene Substanzgemisch wurde aufgearbeitet, wie bei Beispiel 4 beschrieben. Zur Knüpfung der Amidbindung zwischen den Resten 15 und 16 wurden 20 ml Pankreaselastase-Sepharose CL-4B - Beladung: 4 mg Enzym je ml Gel - in 0,05 M Phosphatpuffer, pH 5,2 verwendet. Bei der weiteren Aufarbeitung analog Beispiel 7 wurden 7,1 mg (60 %) farbloses Lyophilisat erhalten.

## Patentansprüche

1. Homologe des Aprotinin, in welchen der Lysinrest im aktiven Zentrum des Inhibitors Aprotinin in Pos. 15 ausgetauscht ist gegen einen der Reste der Aminosäuren Glycin, L-Alanin, L-Valin, L-Leucin, L-Isoleucin, L-Methionin, L-Arginin, L-α-Aminobuttersäure, L-Norvalin, L-Norleucin, Dehydroalanin sowie L-Homoserin.

2. Homologe des Aprotinin, in welchen nur der Lysinrest im aktiven Zentrum des Inhibitors Aprotinin in Pos. 15 gegen einen der Reste der Aminosäuren Glycin, L-Alanin, L-Isoleucin, L-Valin, L-Leucin, L-Arginin, L-α-Aminobuttersäure, L-Norvalin, L-Norleucin, Dehydroalanin sowie L-Homoserin oder deren Alkylester ausgetauscht ist, und deren zusätzlichen Carboxylgruppen in den Seitenketten der sauren Aminosäuren und in terminaler Position gegebenenfalls alle oder teilweise verestert sind.

3. Ein Derivat von Aprotinin, in dem die Peptidbindung zwischen dem Lysin-15-Rest und dem Alanin-16-Rest offen ist, und in dem nur die Carboxylgruppe am Lysin 15 frei vorliegt, alle übrigen Carboxylgruppen in den Seitenketten der Asparaginsäurereste (Pos. 3 und 50) sowie der Glutaminsäurereste (Pos. 7 und 49) und des C-terminalen Alanin (Pos. 58) Alkylestergruppen tragen.

4. Derivat nach Anspruch 3, in dem die Estergruppen Methylester sind.

5. Ein Derivat des Aprotinin mit einer am Alanin-16-gespaltenen Peptidbindung, in dem der Lysin-15-Rest fehlt und die Carboxylgruppen in den Seitenketten der Aspartatreste - 3 und 50 - sowie die Glutamatreste - 7 und 49 - und des C-terminalen Alanin - 58 - Alkylester tragen und lediglich die Carboxylreste am Halbcystinrest 14 unverestert vorliegt.

6. Derivat nach Anspruch 5, in dem die Alkylestergruppe eine Methylgruppe ist.

7. Verfahren zur Herstellung von Homologen des Aprotinin gemäß Anspruch 1, dadurch gekennzeichnet, daß man

a) in Aprotinin die Peptidbindung zwischen Lysin 15 und Alanin 16 spaltet,

b) alle freien Carbonsäuregruppen verestert,

c) gegebenenfalls die Disulfidbrücke zwischen den Cysteinresten 14 und 38 selektiv reduziert,

d) die Estergruppe am Lysin 15 selektiv hydrolysiert,

e) gegebenenfalls die S-S-Brücke zwischen den Cysteinresten 14 und 38 wieder herstellt,

f) den Lysinrest 15 selektiv abspaltet,

g) einen Ester einer der Aminosäuren Glycin, L-Alanin, L-Valin, L-Leucin, L-Isoleucin, L-Methionin, L-Arginin, L-α-Aminobuttersäure, L-Norvalin, L-Norleucin, Dehydroalanin oder L-Homoserin an das Cystein 14 peptidartig ankondensiert,

h) die Peptidbindung zwischen der Aminosäure 15 und Alanin 16 wieder knüpft und

i) die Estergruppen an den freien Carboxylgruppen wieder abspaltet.

j) gegebenenfalls, im Falle einer Kondensation nach Schritt g) mit Carbodiimid, die an den Tyrosinresten entstandenen O-Acylharnstoffgruppen mit Hydroxylamin abspaltet.

8. Arzneimittel, gekennzeichnet durch einen Gehalt an mindestens einem Homologen des Aprotinin gemäß Anspruch 1.

9. Arzneimittel, gekennzeichnet durch einen Gehalt an mindestens einem Homologen des Aprotinin gemäß Anspruch 2.

10. Verwendung von Homologen des Aprotinin gemäß einem der Ansprüche 1 bis 6 zur Herstellung von Arzneimitteln.

## Claims

1. Homologues of aprotinin in which the lysine moiety in position 15 in the active centre of the aprotinin inhibitor has been replaced by one of the moieties of the aminoacids glycine, L-alanine, L-valine, L-leucine, L-isoleucine, L-methionine, L-arginine, L-α-aminobutyric acid, L-norvaline, L-norleucine, dehydroalanine and L-homoserine.

2. Homologues of aprotinin, in which only the lysine moiety in position 15 in the active centre of the aprotinin inhibitor has been replaced by one of the moieties of the aminoacids glycine, L-alanine, L-isoleucine, L-valine, L-leucine, L-arginine, L-α-aminobutyric acid, L-norvaline, L-norleucine, dehydroalanine and L-homoserine or their alkyl esters, and all or some of their additional carboxyl groups in the side-chains of the acidic aminoacids and in the terminal position have, where appropriate, been esterified.

3. A derivative of aprotinin in which the peptide bond between the lysine 15 moiety and the alanine 16 moiety

**0 132 732**

is open and in which only the carboxyl group on the lysine 15 is free, all other carboxyl groups in the side-chains of the aspartic acid moieties (positions 3 and 50) and of the glutamic acid moieties (positions 7 and 49) and of the C-terminal alanine (position 58) bearing alkyl ester groups.

4. Derivative according to Claim 3, in which the ester groups are methyl esters.

5. A derivative of aprotinin having a peptide bond on alanine 16 cleaved, in which the lysine 15 moiety is missing, and the carboxyl groups in the side-chains of the aspartate moieties - 3 and 50 - and the glutamate moieties - 7 and 49 - and the C-terminal alanine - 58 - bear alkyl esters, and only the carboxyl radicals on the half-cystine moiety 14 are non-esterified.

6. Derivative according to Claim 5, in which the alkyl ester group is a methyl group.

7. Process for the preparation of homologues of aprotinin according to Claim 1, characterised in that

a) the peptide bond between lysine 15 and alanine 16 in aprotinin is cleaved,

b) all free carboxylic acid groups are esterified,

c) where appropriate, the disulphide bridge between the cysteine moieties 14 and 38 is selectively reduced,

d) the ester group on lysine 15 is selectively hydrolysed,

e) where appropriate, the S-S bridge between the cysteine moieties 14 and 38 is restored,

f) the lysine 15 moiety is selectively eliminated,

g) an ester of one of the aminoacids glycine, L-alanine, L-valine, L-leucine, L-isoleucine, L-methionine, L-arginine, L-α-aminobutyric acid, L-norvaline, L-norleucine, dehydroalanine or L-homoserine is condensed onto cysteine 14 in the manner of a peptide,

h) the peptide bond between the aminoacid 15 and alanine 16 is formed again and

i) the ester groups on the free carboxyl groups are eliminated again.

j) where appropriate, in the case of condensation according to step g) with carbodiimide, the O-acylurea groups produced on the tyrosine moieties are eliminated using hydroxylamine.

8. Medicament characterised by containing at least one homologue of aprotinin according to Claim 1.

9. Medicament characterised by containing at least une homologue of aprotinin according to Claim 2.

10. Use of homologues of aprotinin according to one of Claims 1 to 6 for the preparation of medicaments.

**Revendications**

1. Homologues de l'aprotinine, dans lesquels le reste lysine du centre actif de l'inhibiteur aprotinine a été remplacé en position 15 par l'un des restes des aminoacides glycine, L-alanine, L-valine, L-leucine, L-isoleucine, L-méthionine, L-arginine, acide L-α-aminobutyrique, L-norvaline, L-norleucine, déshydroalanine ainsi que L-homosérine.

2. Homologues de l'aprotinine, dans lesquels seul le reste lysine du centre actif de l'inhibiteur aprotinine a été remplacé en position 15 par l'un des restes des aminoacides glycine, L-alanine, L-isoleucine, L-valine, L-leucine, L-arginine, acide L-α-aminobutyrique, L-norvaline, L-norleucine, déshydroalanine ainsi que L-homosérine ou par leurs esters alkyliques, et dont les groupes carboxyle supplémentaires, présents dans les chaînes latérales des aminoacides acides et en position terminale, sont éventuellement estérifiés en totalité ou en partie.

3. Dérivé de l'aprotinine, dans lequel la liaison peptidique entre le reste lysine en position 15 et le reste alanine en position 16 est ouverte, et dans lequel seul le groupe carboxyle est libre sur la lysine 15, tous les autres groupes carboxyle des chaînes latérales des restes acide aspariginique (positions 3 et 50) ainsi que les restes acide glutamique (positions 7 et 49) et de l'alanine de carbone terminal (position 58) portant des groupes ester alkylique.

4. Dérivé selon la revendication 3, dans lequel les groupes ester sont des esters méthyliques.

5. Dérivé de l'aprotinine comportant une liaison peptidique scindée sur l'alanine-16, dans lequel manque le reste lysine-15 et les groupes carboxyle des chaînes latérales des restes aspartate - 3 et 50 - ainsi que les restes glutamate - 7 et 49 - et de l'alanine à carbone terminal - 58 - portent les groupes ester alkylique et, seuls les restes carboxyle du reste hemicystine 14 demeurent non estérifiés.

6. Dérivés selon la revendication 5, dans lequel le groupe ester alkylique est un groupe méthyle.

7. Procédé pour préparer des homologues de l'aprotinine selon la revendication 1, caractérisé en ce que

a) on scinde dans l'aprotinine la liaison peptidique entre la lysine 15 et l'alanine 16,

b) on estérifie tous les groupes acide carboxylique libres,

c) on réduit sélectivement, éventuellement, les ponts disulfure entre les restes cystéine 14 et 38,

d) on hydrolyse sélectivement le groupe ester de la lysine 15,

e) on crée éventuellement à nouveau le pont S-S entre les restes cystéine 14 et 38,

f) on scinde sélectivement le reste lysine 15,

g) on fixe par condensation de peptide, sur la cystéine 14, un ester d'un des aminoacides glycine, L-alanine, L-valine, L-leucine, L-isoleucine, L-méthionine, L-arginine, acide L-α-aminobutyrique, L-norvaline, L-norleucine, déshydroalanine ou L-homosérine,

15

h)    on crée à nouveau la liaison peptidique entre l'aminoacide 15 et l'alanine 16, et

i)    on scinde à nouveau les groupes ester sur les groupes carboxyle libres,

j)    éventuellement, en cas d'une condensation, après l'étape g effectuée avec du carbodiimide, on scinde les groupes O-acylurée obtenus sur les restes tyrosine.

8. Médicament, caractérisé en ce qu'il contient au moins l'un des homologues de l'aprotinine selon la revendication 1.

9. Médicament, caractérisé en ce qu'il contient au moins un des homologues de l'aprotinine selon la revendication 2.

10. Utilisation des homologues de l'aprotinine selon l'une des revendications 1 à 6, pour préparer des médicaments.

FIG. 1

FIG. 2